Europäisches Patentamt

European Patent Office

Office européen des brevets

(19)

(11) Publication number: 0 334 539
A2

## (12) EUROPEAN PATENT APPLICATION

(21) Application number: 89302538.7

(22) Date of filing: 15.03.89

(51) Int. Cl.⁴: C12N 15/00 , A01H 1/02 , C12N 13/00

(30) Priority: 21.03.88 GB 8806643

(43) Date of publication of application:
27.09.89 Bulletin 89/39

(84) Designated Contracting States:
AT BE CH DE ES FR GB GR IT LI LU NL SE

(71) Applicant: IMPERIAL CHEMICAL INDUSTRIES PLC
Imperial Chemical House Millbank
London SW1P 3JF(GB)

(72) Inventor: Dunwell, James Martin
3 Hop Gardens
Henley-on-Thames Berkshire RG9 2EH(GB)

(74) Representative: Roberts, Timothy Wace et al
Imperial Chemical Industries PLC Legal
Department : Patents PO Box 6 Bessemer
Road
Welwyn Garden City Herts, AL7 1HD(GB)

(54) Genetic manipulation.

(57) Process for producing plants containing foreign genes which comprises microinjection of transforming DNA into plant embryos through a hole in the cell wall created by a laser.

EP 0 334 539 A2

## GENETIC MANIPULATION

This invention relates to genetic manipulation, and more particularly to the genetic manipulation of plants with the object of providing novel plants, including and expressing desired genes. Such genes may be of the type normally found in the plant species and expressed therein, or they may be genes which are foreign to the plant species or genus, and may for example be genes which are not normally found in or expressed in the plant kingdom at all.

The process of producing a new organism from an existing organism by manipulation of the genome to insert new DNA therein is generally referred to as transformation. A reliable method for the transformation of plants, having wide general applicability, has long been sought. Several methods for the transformation for plants have been proposed, including the use of Agrobacterium tumefaciens, particle bombardment, electroporation and microinjection. None of these have provided a reliable method of broad general application. For example, the Agrobacterium tumefaciens (Ti plasmid) method generally works only with broad leaved (dicotyledonous) species. Particle bombardment, electroporation and microinjection have been made to work in particular cases, but do not seem to be of general applicability. For example, very few monocots have so far been successfully transformed. There is considerable interest in reliable methods of transforming monocots, since so many economically important crops (for example, wheat, maize, barley and rice) are monocotyledonous.

The present invention provides a method of general applicability to the transformation of plants. It can, in principle, be used to transfer any desired DNA into any plant genome, and will in consequence have a very wide range of important possible uses.

According to the present invention we provide a method of producing a transformed plant which comprises forming an aperture in a cell wall of an embryo from the untransformed plant, and introducing genetic material, particularly DNA, into the embryo through said aperture, characterised in that the aperture is formed by means of a laser pulse.

Transformation of animal cells (transfection) has been achieved by such a method (Tsukakoshi, M. et al Appl.Phys. B, 35, 135-140, 1984; EPA 137504) and a machine for this purpose is manufactured by Hitachi. Although a similar machine has been constructed (Wiegand et al, J.Cell Sci., 88, 145-149, 1987), and used in preliminary experiments on plant tissues and cells (Weber et al, Plant Cell Tissue and Organ.Culture, 12, 219-222, 1988) and even organelles (Weber, G., Eur.J.Cell.Biol.,

43, 63 1987, West German Patent application 3707111A) we are aware of no prior proposal to transform plant embryos by this method. This is the more remarkable in view of the widespread interest in reliable methods of transforming plants.

By the term "embryo" as used in the description of the present invention we include the zygote, or an embryo of 2-200 cells derived from the zygote in the course of embryogenesis, or any one or more cells forming part of such an embryo. We also include microspore-derived embryos and somatic embryos containing up to 200 cells. We prefer to use young whole zygotic embryos, in particular embryos containing up to about 20 cells.

The method of our invention may be carried out, in general, by suspending a suitable embryo in a solution, typically an aqueous solution, containing the DNA which it is desired to introduce. Laser apparatus is then focused on a cell of the embryo, and the laser is activated to produce a hole in the wall of the cell. The size of the hole may vary, but must not be too large compared with the size of the cell. In absolute terms, the appropriate size of the hole will differ according to the size of the cell being treated, but will often be between about 5 and about 500 nanometres (nm) in diameter. For some purposes a diameter of between 10 and 100 nm will be appropriate.

The duration of the pulse is conveniently from 5-20 nanoseconds (ns), preferably 10-15 ns. Pulse energy is typically adjusted in the range 1-10 millijoules. More than one pulse may be necessary to penetrate both the cell wall and plasma membrane. Preferably, as many cells as possible in each embryo are injected. Multiple sites of perforation in each cell may be beneficial.

In principle, any laser apparatus capable of being concentrated at an appropriately fine focus may be used in the process of the invention. However, there are already available machines for laser microinjection of mammalian cells. An example is the Hitachi laser microinjector manufactured by Hitachi, Japan and described by Tsukakoshi et al in the Journal of Appl.Phys., B 35, 135-140, 1984 and in EPA 137504. Other laser devices which may be found useful in the process of the invention are described by Wiegand et al, cited above. Wiegand et al use an excimer laser to pump a dye laser, tunable from 217 to 970 nm.

One preferred type of laser is a solid state laser. Such lasers have the advantage of easier control of pulse width, pulse shape and pulse power. Typical wavelengths of such lasers are in the infrared region at 780 nm or 830 nm; but solid state lasers that emit at visible wavelengths or are frequency doubled may also be used.

A further feature of our invention is to include in the solution in which the embryo is suspended a dye having a high extinction coefficient at the wavelength the selected laser emits. For 780 or 830 nm solid state lasers a watersoluble dye such as ICI S116510 infrared dye with an extinction coefficient of 80,000; or the broad band ICI S 109564 with an extinction coefficient of 30,000 may be used. It is preferred to use dyes that bind to the cell wall, eg to the cellulose therein, or to the plasma membrane. By this means improved energy transfer to the plant cell wall and thus more accurate and rapid perforation of the cell wall is obtained. Phytotoxic dyes must be avoided, since they adversely affect the viability of the treated cells.

The embryo is conveniently prepared for treatment by dissection from the plant caryopsis. With the smallest embryos it may be helpful to adopt the method of Norstog, (American J.Bot., 52, 538-546, 1965) which involves the dissection of the end of the caryopsis in a drop of sterile paraffin oil. The isolated embryo is lifted out in the oil film with a capillary and transferred to the nutrient medium. This method prevents drying out of the material during dissection. Caryopsis dissection may be carried out with the assistance of a dissecting or inverted microscope. As embryos are translucent, the use of dark field, Nomarski (DIC) or phase contrast illumination may be helpful.

Following laser treatment, the embryo is allowed to incubate in the DNA solution for a period sufficient to allow diffusion of DNA from the solution into the punctured cell. The incubation period may vary widely, for example from a few seconds to several hours. Generally we find periods of from 5 seconds to 2 hours suitable, and particularly from 1 to 30 minutes. Such incubation is generally carried out at temperatures of from 0°C to 40°C, particularly at temperatures of from 15°C to 30°C, and conveniently at room temperature.

The DNA solution in which the embryo is suspended may comprise from about 0.001 to 2% by weight of the DNA which it is desired to introduce to the cell, together with other ingredients. Such ingredients may be for example inert salts for the purpose of promoting the correct osmotic balance, or cell nutrients, or other additives. The DNA which it is desired to introduce may be in the form of a plasmid or, preferably, linear DNA; it is usually double stranded. The length of the DNA to be introduced may vary from a few bases to hundreds of kilobases. Typically it will comprise a complete heterologous gene with an associated plant-specific control sequence, the gene being one that can be expressed in the plant to be transformed. As an alternative to DNA, the genetic material to be introduced into the cell may be RNA (eg in the form of a retrovirus) that has the power to modify the cell's genome.

The DNA to be introduced may consist of or include one or more of the following types of material:

1. Novel gene or other DNA sequence, conferring new properties on the plant to be transformed. The DNA may encode RNA or protein products, which may be structural, regulatory or catalytic in function. Genes that may be introduced into plants in this way include genes for the production of insecticidal proteins (for example the gene which gives rise to the insecticidal endotoxin found in Bacillus thuringiensis) and genes which confer herbicide resistance.

2. Control sequences located at a suitable distance from and in appropriate orientation to the gene sequence whose expression they are to regulate. The control sequence may include constitutive promoters of transcription, or be responsive to endogenous or exogenous signals - chemical, environmental, temporal, developmental, in response to attack by a pathogen, wounding or the like.

3. Selectable marker genes to permit the identification of transformants by a positive selection procedure. Typically, this would be a gene encoding resistance in transformed plants to an appropriate antibiotic such as Kanamycin or hygromycin. Under some circumstances it may be appropriate to use the novel gene (1 above) for this purpose. Other selectable markers may be desirable to permit the identification of prokaryotic cells that contain the construct.

4. One or more sequences providing additional homology with the target genome; such sequences may provide a higher frequency of homologous recombination and hence more stable transformants. It may also be useful to include such sequences that recombinogenic, that is, that actively promote recombination with the target genome.

5. Sequences required for the propagation of the construct in appropriate bacterial hosts, for example an origin of replication capable of functioning in E. coli.

6. A plant origin of replication (or ARS - autonomously replicating sequence) capable of initiating replication of the introduced DNA in plant cells. This will tend to increase the number of copies to the construct in the target plant cells and thereby increase the probability of achieving a stable transformation event.

After laser treatment and incubation, the resulting embryo, or cells derived from it, are cultured to produce whole plants. Either the individual embryo is allowed to mature into a single plant, or alternatively it may be cloned to produce a number of identical cells each of which can produce a transformed plant. A preferred feature of the invention is to culture the embryo in microdroplets; either in droplets covered in mineral oil according to the method of Schweiger et al, Theor.Appl.Genet., 73, 769-783, 1987), or in 100-1000 nanolitre drops in a polycarbonate microculture chamber according to the method of Spangenberg, G. et al (Physiol Plant., 66, 1-8, 1986), both which descriptions are herein incorporated by reference. Some elements of the culture system are also described by Neuhaus et al, Theor.Appl.Genet., 75, 30-36, 1987). A useful medium for maize embryo culture is described by Burghardtova, K. and Tupy, J., Biol.Plant., 22, 57-64, 1980.

As previously stated, the invention is applicable to a very wide range of plants. Amongst plants that may be transformed are ornamental and crop plants, trees and shrubs. Dicotyledonous plants that may be transformed include tomato, tobacco, sugar beet, rape, cabbage and related Brassica species, potatoes and the like. Monocotyledonous species which may be transformed include onions; grasses, for example ornamental and forage grasses, sugar cane and cereal crops, for example small grain cereals such as wheat, barley, oats, sesame and rice; and corn or maize.

The invention is further described with reference to the following illustrative Example and to the drawings, in which:

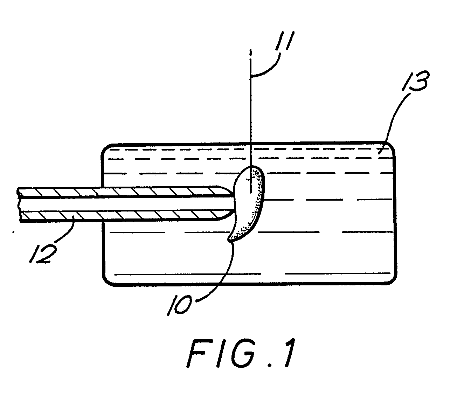

Figure 1 shows schematically the process of embryo injection.


## EXAMPLE 1


### 1. Preparation of Plasmid DNA

The construct for insertion comprises the cauliflower virus (CaMV) promoter, the first intron of the maize alcohol dehydrogenase (ADH) gene, the neomycin phosphotransferase (NPT II) gene, followed by the nopaline synthase (NOS) terminator. This construct is a modification (Callis, J. et al,Genes and Development, 1, 1183-1200, 1987) of that used for the stable transformation of maize protoplasts (Fromm et al, Nature, 319,791-793, 1986). It acts as a kanamycin resistance marker. It is prepared for use by linearisation in known manner, by cleaving at a unique restriction site with the appropriate restriction enzyme, and forming blunt ends to prevent recircularisation.


### 2. Growth of Plants

Maize plants of selected genotype are grown under a controlled environment in a growth room of the type manufactured by Conviron or Weiss. The photoperiod, light intensity and temperature regime are designed to allow healthy growth with normal development of male and female inflorescences (16 hour photoperiod, light intensity 600 $\mu E/m^2/s$, 28°C day/20°C night).


### 3. Isolation of Caryopses

Developing silks are bagged to prevent accidental pollination. Hand pollination is conducted in order to allow strict control over the timing of development of the zygotic embryos (Neuffer, M.G., Maize for Biological Research Ed. W.F. Sheridan, pp. 19-30, 1982). The ear is harvested when the embryo is 10-20 cells in size. This is during the period 3-5 days after pollination and can be calibrated accurately for each genotype. Sterilisation and isolation of individual caryopses is as described by Gengenbach, B.G. (Planta 134: 91-93, 1977; Cell Culture and Somatic Cell Genetics of Plants, Ed. I.K.Vasil pp. 276-282, 1984) or King, P. and Shimamoto, K. (Handbook of Plant Cell Culture, Vol. 2, Eds. W.R. Sharp, D.A. Evans, P.V. Ammirato and Y. Yamada, pp. 69-91, 1984).


### 4. Embryo Preparation

The individual caryopses are dissected under aseptic conditions (King, P. and Shimamoto, K., supra) and the embryos removed by drawing them up into a hand-drawn microcapillary connected to silicone tubing (Neuhaus, G.et al, Theor.Appl.Genet., 75, 30-36, 1987). This operation is conducted with the assistance of a dissecting microscope.


### 5. Embryo Injection

For injection the embryo is transferred to a buffer solution containing the DNA from 1 above (1 $\mu$litre /ml) and with a high osmotic pressure provided by sorbitol (0.4M). The means of injection is a laser mediated system as originally designed for animal cells (Tsukakoshi, M. et al, App.Phys. B., 35, 135-140, 1984) and more recently used for various plant cells (Wiegand, supra; Weber G. et al, 1988, supra). A commercial machine of the necessary type is manufactured by Hitachi and referred to as the Hitachi Laser Microinjector. The pulse duration is 10 ns. Each component cell of the embryo is injected; the embryo is held in a holding pipette during the injection and is rotated to allow access of the beam to each cell. Theprocess is shown schematically in Figure 1, in which 10 is the maize embryo, 11 the laser beam, 12 the holding pipette and 13 the DNA-containing buffer solution.

## 6. Embryo Culture

After injection, the embryo is transferred to culture medium, of the type developed for immature barley embryos by Norstog, K. (In vitro 8, 307-308, 1973) or Jensen, C.J. (Cell and Tissue Culture Techniques for Cereal Crop Improvement, Science Press, Beijing, pp. 55-79, 1983). The embryos are cultured individually, in 15-100nl microdroplets covered by mineral oil (Schweiger, H.G. et al, Theor.Appl.Genet., 73, 769-783, 1987). After 6-10 days in microculture, embryos are transferred to 40 $\mu$l of the same medium in wells of Terasaki dishes (Neuhaus, G. et al, Theor.Appl.Genet., 75, 30-36, 1987). The developing embryos are then transferred (14-28 days) to a Petri dish containing similar medium solidified with agarose or "Gelrite" and containing a reduced content of sucrose (30g/l), in order to stimulate germination. Individual plantlets are then grown further in glass or plastic tubes, still under aseptic conditions.

## 7. Plant Culture and Selection

When approximately 5 to 10cm in height, the plantlets are transferred to compost and grown in the glasshouse, initially under conditions of high humidity and low light levels. Each plant is grown to maturity and carefully
self pollinated. Extreme care is taken to avoid the possibility of contamination of the silk with foreign pollen. The kernels from each plant are carefully harvested. Selection of transformed kernels is carried out by sterilising them and germinating on half strength Murashige and Skoog medium (Physiol.Plant., 15, 473-497, 1962) solidified with agar or "Gelrite". Sections of leaf are isolated from each seedling and incubated on similar medium containing kanamycin or G418. The retention of green colour is an indication of the presence of neomycin phosphotransferase II. Direct assays of enzyme activity are also conducted on tissue sections (Reiss et al Gene, 30, 211-218, 1984; McDonnell et al Plant Mol.Biol.Rep., 5, 380-386, 1987). Seedlings giving a positive reaction to either test are retained and their transgenic nature subsequently confirmed by Southern blotting.

## Claims

1. A method of producing a transformed plant which comprises forming an aperture in a cell wall of an embryo from the untransformed plant and introducing genetic material into the embryo through said aperture, characterised in that the aperture is formed by means of a laser pulse.

2. A method as claimed in claim 1 in which the genetic material is DNA.

3. A method as claimed in claim 2 in which the DNA codes for a heterologous gene.

4. A method as claimed in any of claims 1 to 3 in which the laser is a UV laser.

5. A method as claimed in any of claims 1 to 4 in which employs an excimer laser.

6. A method as claimed in any of claims 1 to 3 in which the laser is a solid state laser.

7. A method as claimed in any of claims 1 to 6 in which the embryo contains from 1 to about 200 cells.

8. A method as claimed in claim 7 in which the embryo is a whole zygotic embryo.

9. A method as claimed in claim 8 in which the embryo is a somatic embryo.

10. Genetically transformed plants obtained as the progeny of embryos treated according to the method claimed in any of claims 1 to 9.

FIG.1